# EUROPEAN PATENT SPECIFICATION

(11) **EP 0 532 646 B1**
(45) Date of publication and mention of the grant of the patent: **10.01.1996**
(21) Application number: 91911457.9
(22) Date of filing: 06.06.1991
(51) Int. Cl.: C07C 11/09, C07C 5/27, C07C 11/08, C07C 2/12, C07C 11/06, C07C 6/04

(54) **PROCESS FOR CATALYTIC CONVERSION OF OLEFINS**
VERFAHREN ZUR KATALYTISCHEN UMSETZUNG VON OLEFINEN
PROCEDE DE CONVERSION CATALYTIQUE D'OLEFINES

(30) Priority: 07.06.1990 GB 9012725
(43) Date of publication of application: 24.03.1993
(73) Proprietor: EXXON CHEMICAL PATENTS INC., Florham Park New Jersey 07932 (US)
(72) Inventor: JANSSEN, Mechilium, Johannes, Gerardus, Clearlake, TX 14423 (US); MORTIER, Wilfried, Jozef, NL-3224 AB Hellevoetsluis (NL); VAN OORSCHOT, Cornelius, Wilhelmus, Maria, NL- 3181 PG Rozenburg (NL); MAKKEE, Michiel, NL-3137 CN Vlaardingen (NL)
(74) Representative: Northover, Robert Frank
(86) International application number: EP9101053
(87) International publication number: WO9118851

(56) References cited:
- EP-A- 0 034 444
- EP-A- 0 111 808
- US-A- 2 217 252

## Description

The present invention relates to a process for conversion of an n-olefin of 3 to 9 carbon atoms to another unsaturated compound. Some unsaturated compounds are readily available e.g. from fossil fuel sources. However the demand for certain unsaturated compounds is greater than for others. There is therefore a need to interconvert unsaturated compounds. In particular, n-olefins may be converted e.g. by skeletal isomerization or by dimerization optionally followed by cracking. Iso-butene is present in the C₄ products obtained from steam-cracking of naphthas or from catalytic cracking of high boiling fuel cuts. Other C₄ products such as n-butene are also present. Demand for iso-butene is increasing and there is therefore a need for a process for converting n-butene efficiently to iso-butene.

Several processes are known for the skeletal isomerization of n-olefins e.g. the production of iso-butene from n-butene. Such conversion has previously been carried out using an amorphous catalyst. For example, EP-A-152918 describes a disproportionation and isomerization catalyst for olefins which comprises silica and molybdenum oxide together with titanium dioxide as promoter.

US 2217252 describes the use of natural and synthetic zeolites such as analcime, chabazite, heulandite, natrolite and thomsonite in the interconversion of olefins.

A process potentially of commercial use for the skeletal isomerization of an olefin is the so-called "Snamprogetti process". In this process the olefin is in contact with an activated alumina catalyst at high temperature (about 450°C) .

The WHSV (weight hourly space velocity) is a measure of the throughput of olefin which is used in continuous use of the catalyst. At 450°C a WHSV of 2 hr⁻¹ is common in the Snamprogetti process. Under these conditions n-butene yields approximately 30% iso-butene with a selectivity of 75%. The catalyst has a life of at least 8 hours before it is necessary to regenerate it.

If the WHSV is increased to more than 12 hr⁻¹ the isobutene yield drops to around 8%.

Another fluorine- or chlorine-containing "activated" alumina catalyst is described in EP-A-0071199. The catalytic activity is comparable with the Snamprogetti catalyst; the isobutene selectivity is higher.

US 4503282 describes the use of AMS-1B boro silicate, a boron containing ZSM-5 type zeolite, in the interconversion of olefins. This catalyst operates at low WHSV (commonly 2 hr⁻¹ or less) and high temperature (400-600°C). The reported selectivities are lower in general than those of the amorphous alumina based catalysts.

Catalysts are also used in the dimerization of n-olefins, but these are not without their problems. It is known to use as a catalyst solid phosphorous acid on kieselguhr. The catalyst cannot be regenerated and disposal of large quantities of catalyst as land-fill material is no longer acceptable.

The applicants sought a catalytic process for the skeletal isomerization or dimerization and optional cracking of olefins, particularly n-olefins of 4 to 6 carbon atoms, which had a good selectivity and yield of the desired olefin for a given WHSV, which had an acceptable catalytic life and which used a catalyst which could be easily regenerated.

Previous research aimed at producing catalysts for the interconversion of unsaturated compounds has been directed to compositions, generally amorphous, which contain Bronsted acid sites i.e. sites which are capable of donating a proton, H⁺. It was believed that interaction between the unsaturated compound and a proton on the surface of the catalyst facilitated the desired conversion.

For example EP 34444 employs a mild steaming treatment which must be very carefully controlled or deactivation will results (page 4 lines 9 to 18). As stated in EP 34444 (claim 1 and page 17 lines 17-21) the zeolite treated must be at least partially in the hydrogen form. This requirement follows the conventional belief that protons are needed to catalyse hydrocarbon conversion reactions. The "acid proton" in a zeolite is part of a structural unit (bridging hydroxyl) which can be depicted as ....... O₃-Si-(OH)-Al-O₃.....

Under the hydrothermal conditions proposed in EP 34444, Al will readily leave the framework. The mechanism for this can be represented by: 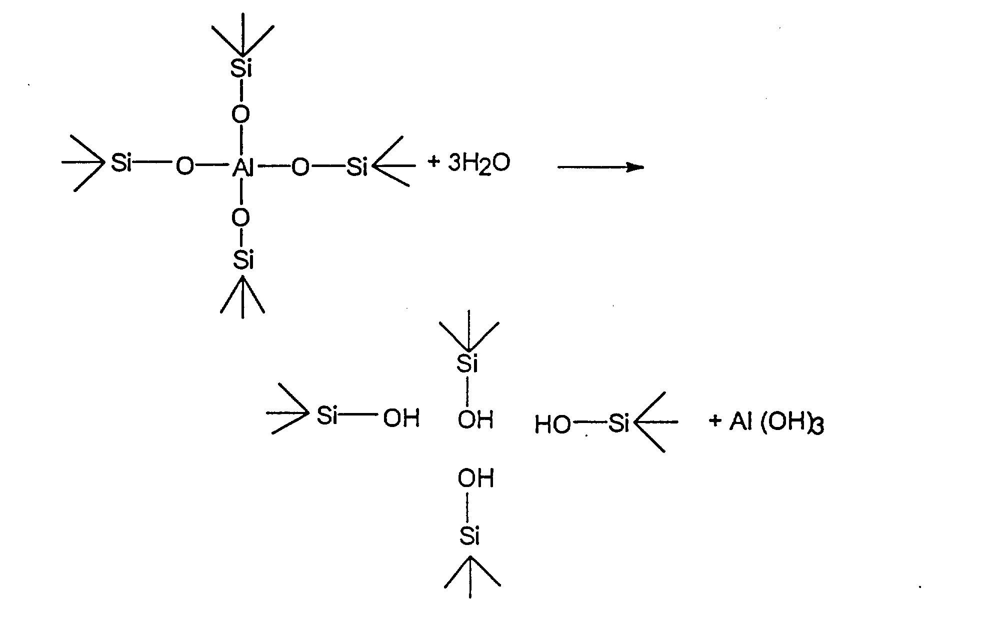 wherby Al(OH)₃ can leave the framework and is exposed at the surface.

In contrast the present invention does not rely on the presence of protons to achieve an improved catalyst. On the contrary the catalyst after ion exchange treatment is substantially free of Broensted acid sites. This approach to improving the catalyst performance avoids weakening the catalyst framework, which will result from the procedures of EP 34444. The catalytic activity of the catalysts of the present invention do not involve cations leaving the framework and thereby the framework weakening because they are not an integral part of the framework anymore. A major advantage of this more stable system is a much longer catalyst life. It is also possible to exercise much greater control of the present invention over the activity of the catalyst than is possible using the condition sensitive process of EP 34444.

The present inventors have surprisingly found that a molecular sieve which has been ion exchanged with a cation to give a Lewis site is a particularly good catalyst for the interconversion of unsaturated compounds.

The present invention provides a process for conversion of an n-olefin of 3 to 9 carbon atoms to another unsaturated compound comprising contacting the olefin in the liquid or vapour phase with a catalyst which comprises a molecular sieve containnig a combination of sites consisting of Lewis acid and buse sites, the Lewis acid sites having been provided by ion-exchanging the molecular have with or cation.

The catalytic process of the present invention has the advantage that it may be used to perform either skeletal isomerization or dimerization and that the dimerization may optionally be combined with cracking. The dominant reaction may be selected by choosing the appropriate reaction conditions bearing in mind the particular catalyst being used.

The catalyst is a molecular sieve, such as a zeolite or a silica/alumina phosphate (SAPO) which should contain a Lewis acid cation site.

The zeolite may be, for example, zeolite Y, zeolite beta, ZK-5, ZSM-5, ZSM-12, ZSM-22 or ZSM-23. Other suitable molecular sieves included SAPO's.

The catalyzed reaction is carried out in the presence of Lewis acid and base sites. This combination of sites may be provided suitably by a molecular sieve. The base sites are present inherently in the molecular sieve e.g. the O⁻ ions in the zeolite framework. Cations present to provide electroneutrality, e.g. divalent cations such as alkaline earth metal cations, provide Lewis acid sites of the desired strength.

It is particularly useful to use as a catalyst a molecular sieve and to ion exchange it with cations. The ion exchange cations present in the sieve do not form an integral part of the framework i.e. they are not covalently bound into the Si/Al/O network of a zeolite. Thus when taking part in the olefin conversion it is not necessary for cations to be removed from the framework and the framework is not weakened. The catalyst composition may be easily controlled by selecting the particular molecular sieve and exchange cations.

In a sieve such as a zeolite the framework is composed of tetrahedra of SiO₄ and AlO₄. The presence of the aluminium produces a charge imbalance in the framework. In a SAPO, some of the (Al,P)O₄ tetrahedra are replaced by (Si,Al)O₄ tetrahedra, which also produces a charge imbalance. To achieve electroneutrality cations are introduced into the sieve. In synthetic zeolites these cations are often alkali metals such as sodium or potassium, or protons.

Such cations may be ion exchanged with other cations which provide stronger Lewis acid sites. Although trivalent cations may be used in the ion exchange the Lewis acid sites produced are generally too strong and it is preferred to use divalent cations. Suitable cations are those whose inclusion in zeolites is known to those of skill in the art, for example, magnesium, calcium, strontium and barium ions. Other divalent cations may also be used to provide Lewis acid strength, for example copper, nickel, and cobalt.

The molecular sieves containing cations will be referred to hereafter by the cation hyphenated to the type of molecular sieve e.g. Mg-beta will be used to denote a zeolite beta containing magnesium ions.

The electronegativity of a zeolite (a function of the composition and the structure type) determines the Lewis acid and base site strength. The electronegativity of a molecular sieve may be varied by methods known to the skilled person, such as ion exchange and/or by changing the Si/Al₂ ratio.

In a typical ion exchange the solid material is mixed with an aqueous solution containing sufficient amount or excess of cation to be introduced. On an experimental scale 10g of solid, for example, may be mixed with 15-25 mls of solution. The mixture is stirred eventually at elevated temperature and the solid is then washed prior to use.

The particular catalyst which will be suitable for any process will depend on the features required for the process. Certain processes will need shorter contact times than others.

When selecting a catalyst for the present process, certain physical attributes of the catalyst should be considered. In general, the higher the ratio of Si/Al₂ the greater the average electronegativity of the catalyst.

A Si/Al₂ ratio of 120 such as may be present in a molecular sieve will result in a higher electronegativity than a Si/Al₂ ratio of 70. A "high" electronegativity tends to lead to a catalyst which is more active or stable than a catalyst of lower electronegativity. A high Si/Al₂ ratio is therefore preferred in the molecular sieve.

The catalyst becomes deactivated during use by the deposition of coke. If the catalytically active sites are far apart then the deactivation may be reasonably slow. Active sites which are far apart i.e. a low active site density are therefore preferred.

One of the factors relating to the density of Lewis acid sites is the cation exchange capacity of the sieve. A sieve which requires the presence of cations for its electroneutrality will have a cation exchange capacity (CEC) generally expressed in milliequivalents/gram (meq/g) for a dried sieve. The higher the CEC, the more closely positioned the active sites for this process. Sieves with a low CEC are therefore preferred in general. The following table gives an indication of CEC values for some of the sieves which may be used in the present process.

| SIEVE | CEC | SiO2/Al203 |
|---|---|---|
| | (meq/g(dried sieve) | |
| Y | 4.53 | 1.16 |
| Mordenite | 2.62 | 2.5 |
| ZSM-12 | 1.72 | 4.17 |
| ZSM-22 | 1.35 | 5.5 |
| ZSM-23 | 1.35 | 5.5 |
| SAPO-11 | 0.04 | - |

When the silica - alumina ratio increases this results in a decrease in the. cation exchange capacity.

Improved stability of catalyst is also seen when the channel dimensions i.e. pore size/void space of the catalyst are reduced. By decreasing the internal volume, coke precursors are prevented from reaching the critical size which would render them unable to leave the pore system. Coke formation is reduced and the deactivation rate of the catalyst will thereby be reduced. If the pore size is too small the catalytic activity is adversely affected. A medium pore size which appears to be the optimum is a T atom ring size of 8 to 12.

A particularly preferred catalyst which has active sites which are far apart, and limited channel dimensions is SAPO-11 i.e. a silica/alumina phosphate which can be synthesized by known methods.

The catalyst may be used in the form of a powder or an extrudate, although the extrudate is usually preferred in industrial processes.

The process of the present invention may be used to isomerize or dimerize and optionally crack n-olefins of 3 to 9 carbon atoms particularly n-olefins of 4 to 6 carbon atoms i.e. n-butene, n-pentene and n-hexene, especially n-butene.

The process may be carried out by charging a reactor such as an stainless steel (SS) reactor with the catalyst and then activating the catalyst. This is generally achieved by continuously passing an inert gas such as nitrogen through the catalyst for 1-3 hours at elevated temperature. The temperature and pressure in the reactor are then adjusted to the conditions under which it is desired to carry out the reaction. The olefin is then fed through the catalyst, optionally with an inert diluent.

When the reaction temperature is in the range 250-500°C especially 375-475°C and the reaction pressure is 0.08-0.12 MPa, especially atmospheric pressure, the major reaction will be the skeletal isomerization of the n-olefin. Thus n-butene will be isomerized to give iso-butene. At higher temperatures the secondary C₄ reactions such as alkylation, dimerization, hydrogenation and cracking are suppressed but the formation of the iso-butene is also less. The above ranges are believed to provide the optimum amount of product with acceptably small levels of side products.

Reducing the temperature and increasing the pressure will tend to favour formation of the dimerized products. When the reaction temperature is 80-250°C, especially around 200°C, and the reaction pressure is relatively high e.g. greater than 10 bar g, the major reaction will be the dimerization of the n-olefin. Thus n-butene will be dimerized to give unsaturated C₈ compounds.

On the other hand if the reaction temperature is maintained at a temperature comparable with that used for isomerization i.e. 250-500°C but the pressure is increased from isomerisation pressure to 0.12-2 MPa, this favours the formation of disproportionated dimerization products; for example n-butene under these reaction conditions will form propene and pentenes.

Using the present process the skilled person can control the form of catalyst used by selecting a particular molecular sieve structure and pore size and ion exchanging the sieve with the desired cation to provide Lewis acid sites in the sieve. Once the catalyst has been selected, the reaction conditions appropriate for the desired product using that catalyst can be established by adjusting the temperature and pressure based on the knowledge of which products are favoured by increasing or decreasing the temperature or pressure.

The catalyst will eventually require regeneration to rid it of the coke deposits which gradually build up the pores. The regeneration may be easily carried out by a coke burn in air at a temperature of 350-700°C, preferably 450-550°C.

The following examples illustrate the invention.

### EXAMPLE 1 - Skeletal isomerization

A stainless steel (SS) reactor was charged with 1.0 g of catalyst, particle size 210 µm < d < 600 µm.

Prior to use the catalyst was activated at 500°C for 2 hours under a continuous flow of nitrogen (50 ml/min). After activation the reactor temperature was changed to the desired temperature.

In all experiments 1-butene was used as a feed. If, necessary the feed stream was diluted with nitrogen.

The reactions were run for different times, depending on the deactivation rate of the catalyst.

Samples of the product stream were analyzed every hour by gas chromatography (GC) on line, using an Al₂O₃/KCl plot column.

The following test conditions were used:

| | |
|---|---|
| Temperature : | 375°C |
| Pressure : | atmospheric |
| WHSV : | 1 |
| HC/Diluent : | 1 : 3 |
| Diluent : | nitrogen |

The results are shown in Tables I and II.

The time on stream taken to reach a conversion level of 10% can be used to measure the stability of the catalyst; the longer this time, the more stable the catalyst.

With decreasing Lewis acid strength (in the order of Mg(II), Ca(II), Sr(II) and Ba(II)), the following observations were made:
- increase in isobutene selectivity;
- increase in catalyst life;
- increase in the heavies selectivity;
- decrease in lights selectivity and
- decrease in hydrogen transfer (ratio of C4 and C3=).

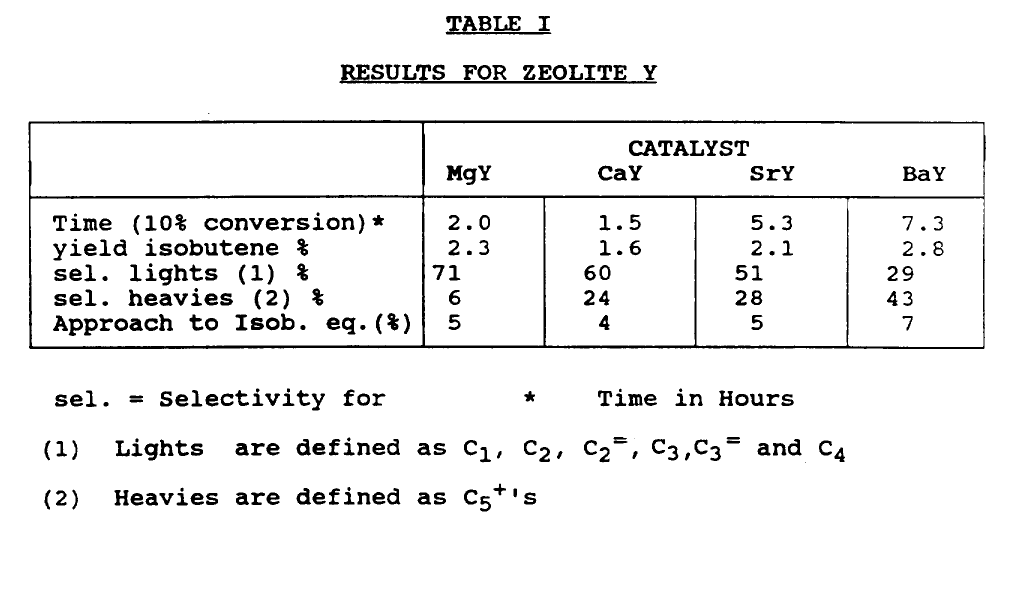

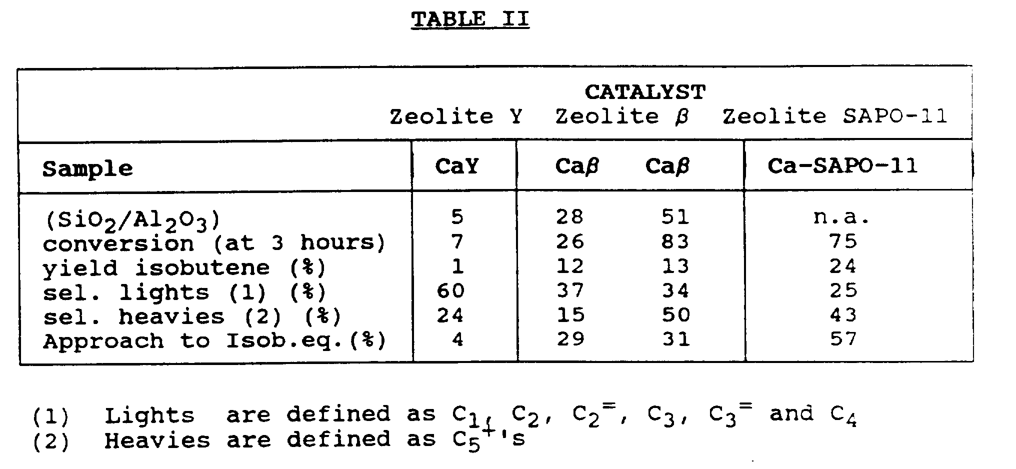

The deactivation rate of zeolite Y is high. It is known from the open literature that coke formation increases with the radius of the zeolite channels and cages. Zeolite Y is one of the larger pore zeolites, therefore showing a fast deactivation. Zeolite Beta in comparison with zeolite Y, has fewer acid sites and also has smaller channels.

A comparison of the results for Ca-Beta and Ca-Y shows that smaller zeolite channels and lower acid site density resulted in a lower deactivation rate and a higher isobutene selectivity. For Ca-Beta with SiO₂/Al₂O₃ ratio of 51 the acid site density is lower than that of Ca-Beta with an SiO₂/Al₂O₃ ratio of 28. Therefore, the deactivation rate of Ca-Beta(51) is lower and the isobutene yield is higher than that of Ca-Beta(28). For the SAPO-11 molecular sieves, the acid site density is very low and the channel radius is smaller than for zeolite Y and zeolite Beta. The results for Ca(II) exchanged SAPO-11 as indicated in Table II showed an isobutene yield of 24 wt % and a very low deactivation rate.

Thus the smaller pores in zeolite Beta in comparison with zeolite Y led to a lower deactivation rate of the catalyst, i.e. a suppression of side reactions.

The even lower deactivation rate of the SAPO-11 catalyst was also due to the small pore size and also the isolated acidic sites.

Varying the WHSV does not affect the initial activity or selectivity of the catalyst. However, deactivation is affected by WHSV; the lower the WHSV, the longer the catalyst life.

### Regeneration

The catalysts were each regenerated by treating with 5% oxygen in nitrogen at 550°C. When re-used in the isomerization process they showed the same initial activity/deactivation rate but a slight change in selectivities. The Ca-SAPO-11 selectivity for isobutene was slightly down (31 to 28), for lights was slightly lower (24 to 18) and for heavies slightly up (45 to 54).

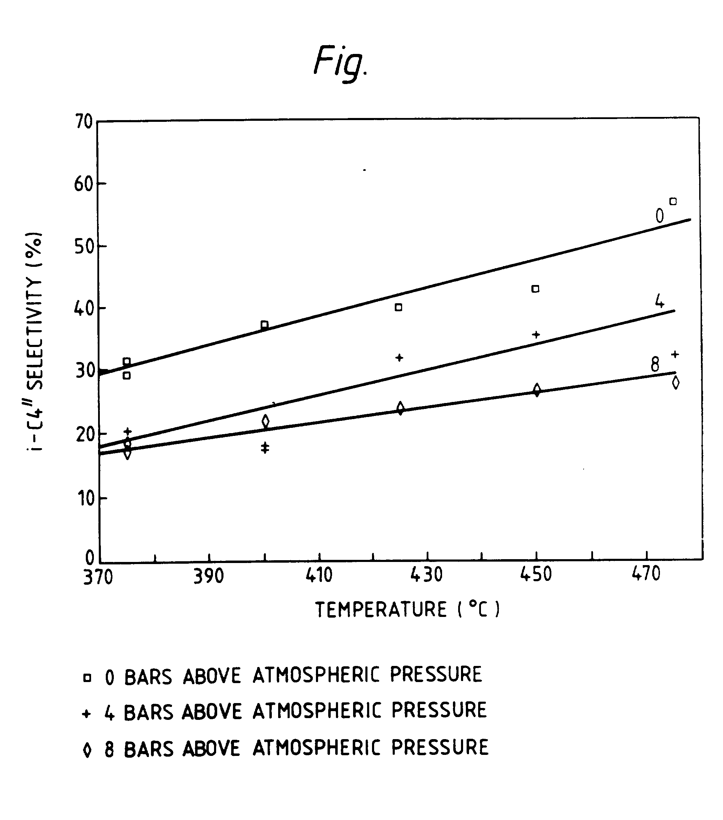

The Figure shows the variation in the selectivity for isobutene with varying temperature and pressure using a Ca-SAPO-11 catalyst. It can be seen from this that high temperatures and low pressures favour production of the skeletally isomerized product.

### EXAMPLE 2 - Skeletal isomerization using SAPO-11 extrudates.

Commercially available SAPO-11 extrudates (as received, Mg(II)- and Ca(II) exchanged forms) were tested for the skeletal isomerization of n-butenes. In all experiments the selectivity for isobutene is somewhat higher than in the powder experiments. The deactivation rate for the extrudates is slightly higher. The time required to reach the maximum yield of isobutene is 24 hours for the Mg(II)- and Ca(II) exchanged extrudates, 48 hours for the as received extrudates and 1 - 2 hours for the powders. The catalyst life of the SAPO-11 extrudates is over 140 hours run length and the isobutene selectivity is around 125 % of the thermodynamic equilibrium. The high yield of isobutene over molecular sieve catalysts (125 % of thermodynamic equilibrium) can be explained as follows: 1) one part of isobutene is formed via the skeletal isomerization and 2) the other part of isobutene is formed via a dimerization of butenes to octenes and followed by selective cracking of these octenes to propene, pentenes and especially isobutene (and other butenes). The results are shown in Table III.

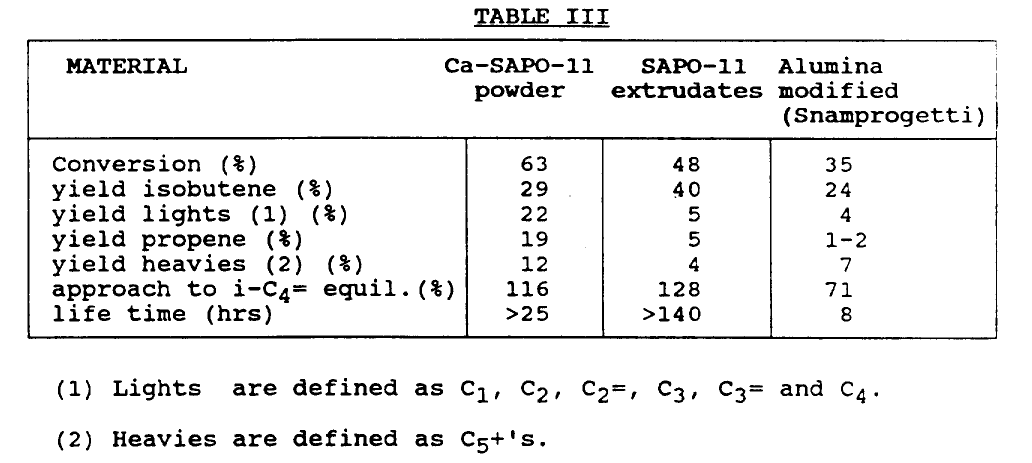

### EXAMPLE 3 - Production of Propene using SAPO-11 extrudates.

A SS reactor was charged with 1.0 gram of commercially available SAPO-11 extrudates (as received, Ca(II) exchanged form), particle size 210 µm <d< 600 µm. Prior to use the catalyst was activated at 500°C for 2 hours under a continous flow of dried nitrogen (50 ml/min). After activation the reactor temperature was lowered to the desired temperature.

In the experiments 1-butene was used as a feed, diluted with nitrogen. The pressure drop over the reactor was < 0.1 barg. Product analysis was done by gas chromatography on line, using an Al₂O₃/KCl plot column.

The following test conditions were used:

| | |
|---|---|
| Temperature | : 475°C |
| Catalyst | : Ca-SAPO-11 extrudates |
| WHSV | : 1 |
| HC/Diluent | : 1:3 |
| Diluent | : nitrogen |

The results are shown in Table IV.

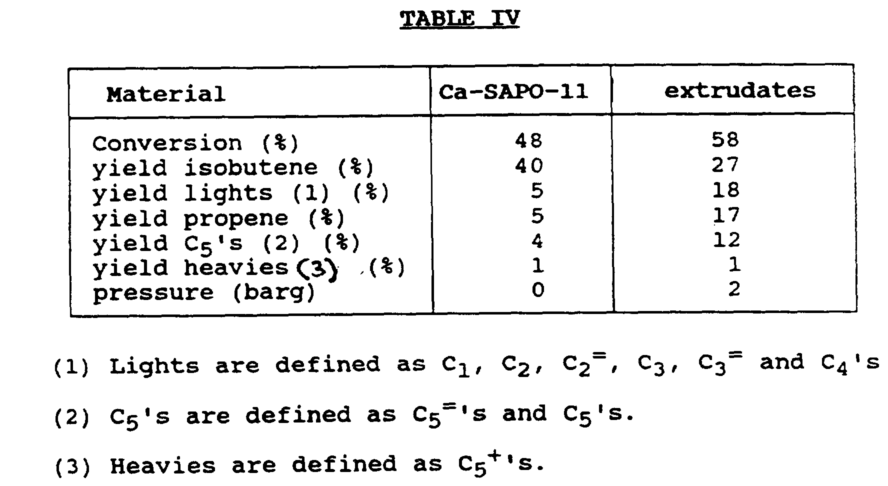

An increase in pressure results in selective cracking of the dimerisation-formed octenes into the valuable propene and pentenes.

### EXAMPLE 4 - Dimerization

The dimerization of l-butene was studied in a stainless steel fixed bed reactor. The temperature was 200°C, pressure was 10 barg and WHSV of 1.5. Prior to use the catalysts were dehydrated and activated at 500°C for 16 hours under dried air at a flow rate of 1000 (ml/ml(cat) x hrs). The pressure drop over the reactor was in all experiments <0.1 barg. Product analysis was done by gas chromatography using a squalane column with hydrogen as carrier gas. The average carbon number distribution number was determined off-line with gas chromatography using a 13X molecular sieve column. For both GC analyses, the sample was hydrogenated in situ in the injector system of the GC over a platinum on alumina catalyst in order to simplify the analysis, by hydrogenation of the olefins into paraffins. Various types of catalyst/zeolites were tested: molecular sieves SAPO-11 extrudates (obtained from Union Carbide, crystal size distribution 0.5 - 5 µm (main distribution 1.3 -2.2 µm)) and zeolite Beta (silica-alumina ratio of 51). The catalyst extrudates were crushed to a particle size distribution from 0.2-0.6 mm in order to eliminate diffusion phenomena and reactor wall effects.

The results are shown in Tables V to VIII.

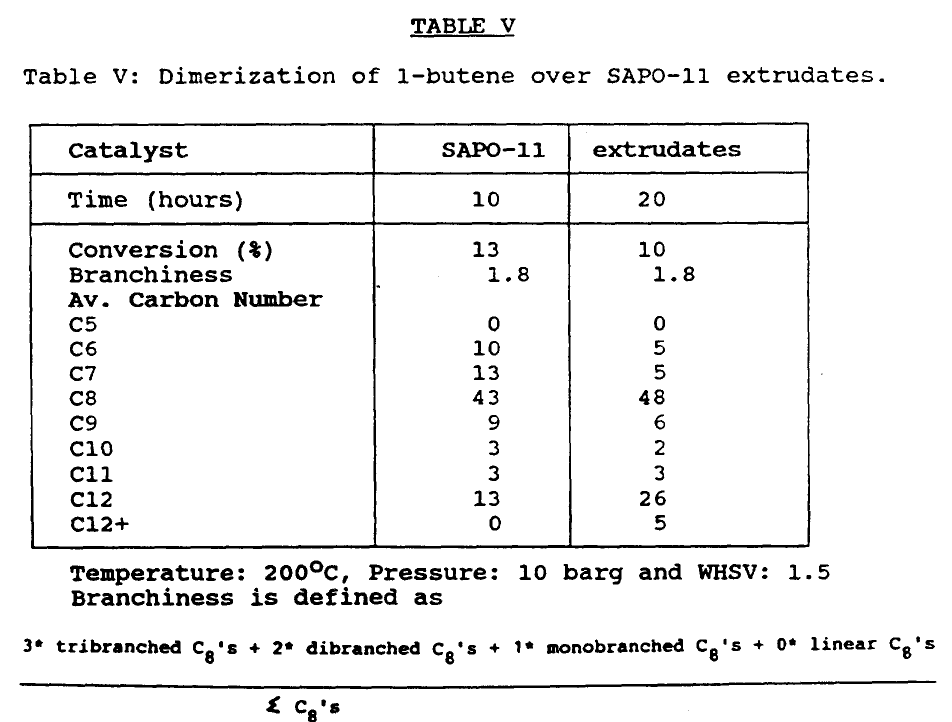

The random carbon number distribution and the residues on the catalyst indicate that the overall acidity of the SAPO-11 is high (high oligomerization and cracking rates). Cation exchange of zeolites/molecular sieves can tailor the strength of the acidity to produce catalysts for the present process.

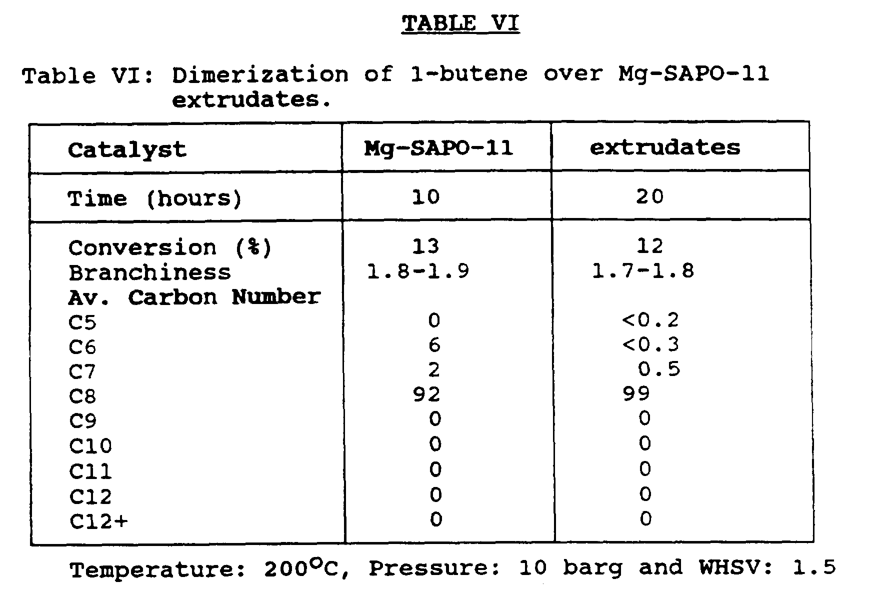

As can be seen in Table VI, the activity of a magnesium exchanged SAPO-11 extrudates catalyst is the same as for the untreated SAPO-11 extrudates. The branchiness is 1.6. The selectivity to the C8's is exceptionally high (>98%).

For the dimerization reaction of butenes over a non-ion exchanged zeolite catalyst the presence of the transition metal nickel its oxidation state on the catalyst support is essential. SAPO-11 extrudates as received were exchanged with nickel and subsequently in situ in the reactor activated at 500°C for 16 hours under dried air flow. The nickel exchange level was not analyzed.

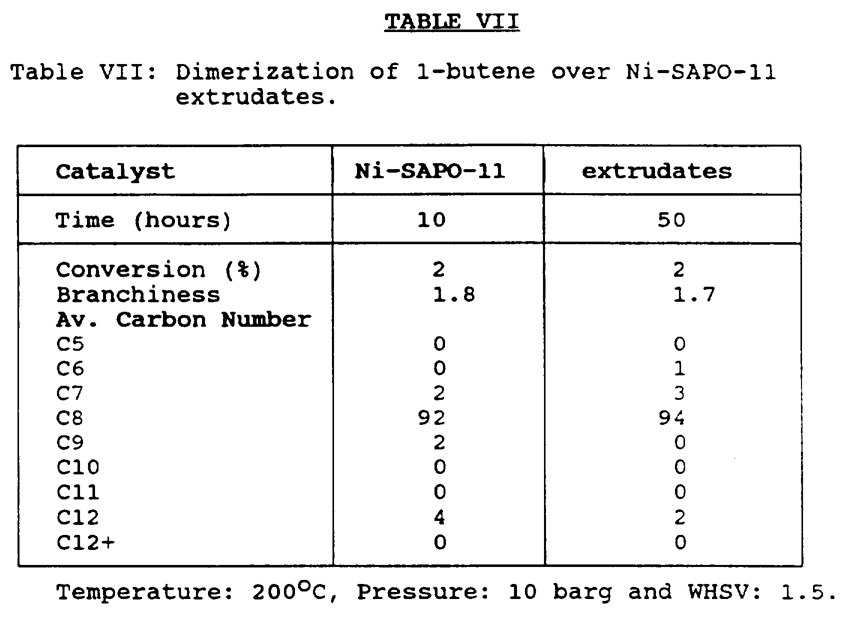

As can be seen in Table VII the activity of the nickel exchanged SAPO-11 extrudates is, in comparison with the previously tested catalysts (SAPO-11 and Mg-SAPO-11), very low.

The activity maintenance of this catalyst is good. Even up to 100 hours on stream the conversion level remains unchanged (2%). The selectivity to C8's and the branchiness of this fraction are comparable to those of Mg-SAPO-11 (>92% and 1.7-1.8, respectively).

In zeolite Beta the number of cation exchangeable sites is higher than in SAPO-11 and it might be expected that nickel exchanged zeolite Beta would be more active. Nickel exchanged zeolite Beta was activated in situ in the reactor at 500 C for 16 hours under dried air. The nickel exchange level was not analyzed.

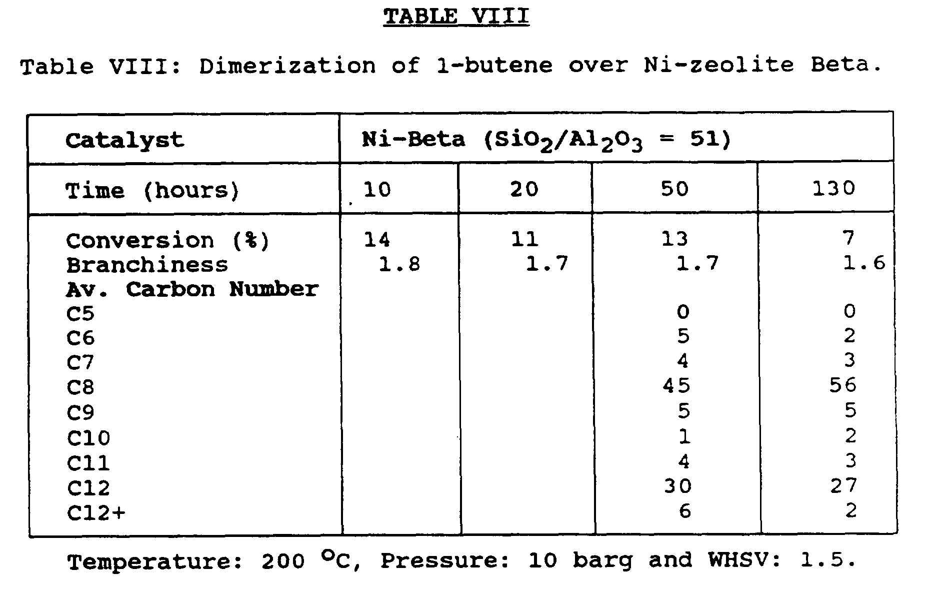

As can be seen in Table VIII the activity of nickel exchanged zeolite Beta is much higher (14% conversion versus 2% for Ni-SAPO-11). Although the initial conversion level drops over the first 20 hours, the conversion level remains unchanged up to 130 hours on stream (still 7 % conversion up to 130 hours on stream). The branchiness of the C8 fraction is good (1.6-1.8). After the run on the catalyst some amounts of C18 and C22 were observed. The random carbon number distribution and the amounts of residues on the catalyst indicate that the overall acidity of the Beta is rather high (high oligomerization and cracking rates).

## Claims

1. A process for conversion of an n-olefin of 3 to 9 carbon atoms to another unsaturated compound comprising contacting the olefin in the liquid or vapour phase with a catalyst which comprises a molecular sieve containing a combination of sites consisting of Lewis acid and base sites, the Lewis acid sites having been provided by ion exchanging the molecular sieve with a cation.

2. A process according to claim 2 in which the molecular sieve is a zeolite or a silica/alumina phosphate (SAPO).

3. A process according to claim 2 in which the molecular sieve is zeolite Y, zeolite Beta, ZK-5, ZSM-5, ZSM-12, ZSM 22, ZSM-23 or a SAPO-11.

4. A process according to any one of the preceding claims in which the Lewis acid sites are provided by a divalent cation.

5. A process according to claim 4 in which the cation is Mg, Sr or Ba.

6. A process according to any one of the preceding claims in which the catalyst is a SAPO-11 containing Mg or Ca ions.

7. A process according to any one of the preceding claims in which the n-olefin is n-butene, n-pentene or nhexene.

8. A process according to any one of the preceding claims in which the n-olefin is n-butene which is skeletally isomerized to iso-butene or dimerized to give octenes or converted to propene and pentenes.

9. A process according to claim 8 which is an isomerization carried out at a temperature of 250 to 500°C and pressure of 0.08-0.12 MPa.

10. A process according to claim 8 which is a dimerization carried out at a temperature of 80-250°C and pressure of greater than 10 MPa.

11. A process according to claim 8 which is a conversion to propene and pentenes carried out at a temperature of 250-500°C and pressure of 0.12-2 MPa.

## Patentansprüche

1. Verfahren zur Umwandlung eines n-Olefins mit 3 bis 9 Kohlenstoffatomen in eine andere ungesättigte Verbindung, bei dem das Olefin in der flüssigen oder Dampfphase mit einem Katalysator kontaktiert wird, der ein Molekularsieb umfaßt, das eine Kombination von Stellen enthält, die aus Lewissäure- und -basestellen bestehen, wobei die Lewissäurestellen durch Ionenaustausch des Molekularsiebs mit einem Kation geliefert worden sind.

2. Verfahren nach Anspruch 2, bei dem das Molekularsieb ein Zeolith oder ein Siliciumdioxid/Aluminiumoxid-Phosphat (SA-PO) ist.

3. Verfahren nach Anspruch 2, bei dem das Molekularsieb Zeolith Y, β-Zeolith, ZK-5, ZSM-5, ZSM-12, ZSM 22, ZSM-23 oder SAPO-11 ist.

4. Verfahren nach einem der vorhergehenden Ansprüche, bei dem die Lewissäurestellen von einem zweiwertigen Kation geliefert werden.

5. Verfahren nach Anspruch 4, bei dem das Kation Mg, Sr oder Ba ist.

6. Verfahren nach einem der vorhergehenden Ansprüche, bei dem der Katalysator ein SAPO-11 ist, der Mg- oder Ca-Ionen enthält.

7. Verfahren nach einem der vorhergehenden Ansprüche, bei dem das n-Olefin n-Buten, n-Penten oder n-Hexen ist.

8. Verfahren nach einem der vorhergehenden Ansprüche, bei dem das n-Olefin n-Buten ist, das zu Isobuten skelettisomerisiert wird oder unter Bildung von Octenen dimerisiert wird oder in Propen und Pentene umgewandelt wird.

9. Verfahren nach Anspruch 8, das eine bei einer Temperatur von 250 bis 500°C und einem Druck von 0,08 bis 0,12 MPa durchgeführte Isomerisierung ist.

10. Verfahren nach Anspruch 8, das eine bei einer Temperatur von 80 bis 250°C und einem Druck von mehr als 10 MPa durchgeführte Isomerisierung ist.

11. Verfahren nach Anspruch 8, das eine bei einer Temperatur von 250 bis 500°C und einem Druck von 0,12 bis 2 MPa durchgeführte Umwandlung zu Propen und Pentenen ist.

## Revendications

1. Procédé pour la conversion d'une n-oléfine de 3 à 9 atomes de carbone en un autre composé insaturé, comprenant la mise en contact de l'oléfine en phase liquide ou en phase vapeur avec un catalyseur qui comprend un tamis moléculaire contenant une association de sites consistant en sites acides de Lewis et sites basiques de Lewis, les sites acides de Lewis ayant été engendrés en incorporant un cation au tamis moléculaire par échange d'ions.

2. Procédé suivant la revendication 1, dans lequel le tamis moléculaire est une zéolite ou une association silice/phosphate d'alumine (SAPO).

3. Procédé suivant la revendication 2, dans lequel le tamis moléculaire consiste en zéolite Y, zéolite bêta, ZK-5, ZSM-5, ZSM-12, ZSM 22, ZSM-23 ou SAPO-11.

4. Procédé suivant l'une quelconque des revendications précédentes, dans lequel les sites acides de Lewis sont engendrés au moyen d'un cation divalent.

5. Procédé suivant la revendication 4, dans lequel le cation consiste en Mg, Sr ou Ba.

6. Procédé suivant l'une quelconque des revendications précédentes, dans lequel le catalyseur consiste en un SAPO-11 contenant des ions Mg ou Ca.

7. Procédé suivant l'une quelconque des revendications précédentes, dans lequel la n-oléfine consiste en n-butène, n-pentane ou n-hexane.

8. Procédé suivant l'une quelconque des revendications précédentes, dans lequel la n-oléfine consiste en n-butène dont le squelette est isomérisé en isobutène ou dimérisé en donnant des octènes ou bien transformé en propène et pentènes.

9. Procédé suivant la revendication 8, qui est une isomérisacion effectuée à une température de 250 à 500°C et sous une pression de 0,08 à 0,12 MPa.

10. Procédé suivant la revendication 8, qui est une dimérisation effectuée à une température de 80 à 250°C et sous une pression supérieure à 10 MPa.

11. Procédé suivant la revendication 8, qui consiste en une conversion en propène et pentènes effectuée à une température de 250 à 500°C et sous une pression de 0,12 à 2 MPa.
